# FASCICULE DE BREVET EUROPEEN

(11) **EP 0 859 011 B1**
(45) Date de publication et mention de la délivrance du brevet: **02.05.2001**
(21) Numéro de dépôt: 98400322.8
(22) Date de dépôt: 12.02.1998
(51) Int. Cl.: C08B 11/145, C08L 1/02, C09K 7/02, C04B 7/00, C02F 1/56, D21H 17/26, D21H 19/34, C09D 7/02, A61K 7/00

(54) **Procédé pour obtenir des microfibrilles de cellulose cationiques ou leurs dérivés solubles, ainsi que celluloses obtenues par ce procédé**
Verfahren zur Herstellung von kationischen Zellulosemicrofasern oder deren löslichen Derivaten und Zellulose hergestellt durch dieses Verfahren
Process for obtaining cationic cellulose microfibrils or their soluble derivatives as well as celluloses obtained by this process

(30) Priorité: 12.02.1997 FR 9701623
(43) Date de publication de la demande: 19.08.1998
(73) Titulaire: AGRO INDUSTRIE RECHERCHES ET DEVELOPPEMENTS (A.R.D.), 51110 Pomacle (FR)
(72) Inventeur: Ralainirina, Robert, 51100 Reims (FR); Rinaudo, Marguerite, 38000 Grenoble (FR); De Baynast, Régis, 78000 Versailles (FR); Desbrieres, Jacques, 38500 Voiron (FR)
(74) Mandataire: Hammond, William

(56) Documents cités:
- EP-A- 0 051 230
- EP-A- 0 120 471
- EP-A- 0 310 787
- WO-A-92/19652
- CHEMICAL ABSTRACTS, vol. 123, no. 24, 11 décembre 1995 Columbus, Ohio, US; abstract no. 316739, "Cationization of cellulose fabric using *-chloro-2-hydroxypropyltriethyl ammonium chloride" XP002043896 & KIM Y. J. ET AL.: HAN'GUK SOMYU KONGHAKHOECHI, vol. 32, no. 6, 1995, S. KOREA, pages 562-569,

## Description

La présente invention est relative à un procédé pour obtenir des microfibrilles de cellulose cationiques ou leurs dérivés solubles, ainsi qu'aux celluloses obtenues par ce procédé.

L'obtention de microfibrilles et leurs applications sont en particulier décrites dans les brevets américains n° 4.374.702, 5.037.334, 4.341.807 et 4.278.381. Les microfibrilles obtenues à partir de cellulose sont connues pour leurs propriétés épaississantes : elles trouvent par suite des applications dans le domaine de l'alimentation humaine ou animale, des cosmétiques et de la chimie. Cependant, l'aspect des microfibrilles de cellulose telles qu'obtenues selon l'art antérieur, est très hétérogène, ce qui, par exemple, rend leur emploi très difficile pour la formulation.

De plus, la réhydratation de ces microfibrilles de l'art antérieur après séchage ou déshydratation se révèle actuellement impossible, d'où une importance des coûts de transport. Du fait de la présence d'eau en quantité non contrôlable, de telles microfibrilles ne peuvent être utilisées en cosmétique, en agro-alimentaire, en papeterie, etc.

Certains éléments de charge ont été utilisés pour limiter la formation de tels agrégats, lors de la déshydratation, comme par exemple des tensioactifs cationiques et d'amide d'acides gras quaternisé (cf. US-A-4.144.122). Certes, le document US-A-4.481.077 met en évidence la possibilité d'améliorer l'utilisation de cellulose déshydratée par l'adjonction d'agents de charge comme les oligo- ou polysaccharides, l'amidon, le glycol, etc. Cependant, ceci ne suffit pas pour retrouver la qualité physico-chimique de la cellulose et en particulier les propriétés rhéologiques. En effet, un nouveau passage dans l'homogénéisateur haute pression est nécessaire.

Des réactions de déshydratation ont été réalisées par lyophilisation et permettent de garder les propriétés épaississantes des microfibrilles. Si cette technique semble efficace (US-A-4.474.949), le procédé est particulièrement long et coûteux et ne garantit pas de retrouver la viscosité du produit de départ car le réseau fibrillaire est abîmé lors de la formation des cristaux. (SACHS, I.B. 1985 Preserving and recovering pulp fibrils subsequent to drying. Paper and industry 38-41).

Seules les techniques dites de point critique de séchage permettent d'éviter ce phénomène, notamment en remplaçant un solvant (ex. : éthanol) par un fluide de transition (ex. : CO₂ liquide). On joue alors sur les températures et pressions de deux solvants se trouvant en phase gazeuse ou liquide. Cette technique est particulièrement onéreuse et compliquée à mettre en oeuvre et ne permet pas de retrouver complètement les propriétés initiales du produit (tout au plus 80 %).

La réaction de cationisation des polysaccharides en général, de quaternisation en particulier, a été beaucoup étudiée dans la littérature et surtout appliquée à la molécule d'amidon. L'amidon cationique obtenu, selon le degré de substitution (DS) visé, est utilisé soit en surfaçage (amidon cationique de DS 0,01 à 0,03), soit dans la masse (amidon cationique de DS 0,04 à 0,07). Ces composés trouvent leurs applications en tant qu'additifs dans la fabrication du papier.

En effet, la réaction conduit en général à des composés cationiques de degré de substitution relativement faible de l'ordre de 0,01 à 0,09 (voir DE-A-3.644.579, US-A-3.448.101, O.B. Wurzburg « Modified starches : properties and uses » édition 1986), bien que celle-ci soit réalisée en présence d'un fort excès d'agents cationiques et d'un catalyseur basique.

Une grande diversité de dérivés de polysaccharides, notamment d'amidon et de cellulose, ayant des degrés de substitution variables ont été décrits dans l'art antérieur. Toutefois, les divers procédés et les diverses conditions de traitement décrits pour préparer ces dérivés donnent des produits ayant généralement un degré de substitution inférieur à 0,1 environ et le plus souvent inférieur à 0,05, ou nécessitent l'utilisation de solvants organiques qui rendent le procédé coûteux, ou impliquent certains procédés de manipulation peu commodes et/ou polluants.

La réaction de quaternisation peut être effectuée selon deux méthodes : la première est réalisée par la voie classique à savoir la voie humide généralement en phase aqueuse, et la seconde voie consiste à effectuer la réaction par voie sèche. Ces deux approches nécessitent l'utilisation d'un important excès de réactifs cationiques. En effet, lorsque la réaction est mise en oeuvre en milieu aqueux, une majeure partie du réactif est hydrolysée et ne peut plus réagir sur le substrat. Quant à la seconde voie, elle ne permet pas un bon contact entre l'agent cationique et le substrat : la réaction se fait uniquement en surface des fibres de cellulose. A titre d'exemple, pour un degré de substitution visé de 0,05, il faut utiliser 0,39 moles d'agents cationiques ce qui veut dire qu'on utilise 7,8 fois plus de réactifs cationiques que la stoechiométrie, autrement dit la perte s'élève à plus de 87 % de réactif (en sachant qu'un DS de 0,05 correspond à une consommation de 0,05 moles environ de réactif).

En outre, la voie sèche nécessite un temps de réaction plus long (8 à 10 jours) et donc immobilise des réacteurs.

Pour ce qui est des polymères naturels modifiés par quaternisation ayant un degré de substitution allant de 0,2 à 0,7, la plupart des travaux décrits dans la littérature portent là encore principalement sur l'amidon (cf US-A-3.842.005, US-A-3.875.054, US-A-3.835.114, US-A-3.962.079).

Concernant la modification de la cellulose par substitution chimique, elle a été également largement étudiée. On peut citer tout d'abord les esters de cellulose tels que les acétates de celluloses et les esters aliphatiques de cellulose. Ces composés de degré de substitution compris entre 1,5 et 3 sont synthétisés pour en faire des matériaux plastiques biodégradables et leur mode de préparation nécessite le plus souvent l'utilisation de solvants organiques toxiques et polluants tels que la pyridine et le chloroforme et d'un excès important d'agents estérifiants (voir C.J. Malm J.W. Mench, D.L. Kendall, et G.D. Hiatt « Industrial and Engineering Chemistry vol. 43 1950 684, H.S. Kwatra, J. M. Caruthers et B.Y. Tao » Ind. Eng. Chem. Res. vol. 32 1992, 2647-2651).

Il y a également les éthers de cellulose. Les dérivés éthers les plus connus sont la carboxyméthylcellulose (CMC), l'hydroxyéthylcellulose (HEC), l'hydroxypropylcellullose (HPC) ou la méthylcellulose (MC) pour ne citer que ceux-là. Leur mode de synthèse ainsi que leurs applications (que ce soit en alimentaire ou dans le domaine de la cosmétique) ont fait l'objet de nombreuses publications tant dans des revues scientifiques que dans des brevets. La préparation de la plupart de ces éthers de cellulose nécessite également l'utilisation de solvants organiques tels que le toluène ou le dichlorométhane ; et parfois un prétraitement en milieu alcalin de la cellulose est nécessaire afin de rendre accessible les fonctions hydroxyles libres (voir Methods in carbohydrate chemistry vol. III « Cellulose » par Roy L. Whistler, édition Academic Press 1963 ; DE-A-433 5437, JP-A-05877001, JP-A-05017501).

Certains de ces composés sont depuis longtemps sur le marché. On peut citer entre autre la carboxyméthylcellulose (CMC) commercialisée par Dow Chemical Company, utilisée en tant qu'épaississant, émulsifiant, agent stabilisateur d'émulsions ou encore en tant qu'émolliant. En alimentaire, la CMC est utilisée comme agent filmogène.

Ces dérivés éthers de cellulose ont plus ou moins les mêmes propriétés et les mêmes fonctions et présentent tous des caractéristiques similaires (solubles dans l'eau, plus ou moins solubles dans l'éthanol selon leur degré de substitution). Toutefois, ils ont des comportements différents en fonction du pH et en présence des sels. En effet, les dérivés non ioniques restent relativement stables dans une zone de pH comprise entre 3 et 11, tels que les méthylcelluloses commercialisées par Dow Chemical Company sous la marque Methocel. Quant aux sels, ils ont une influence sur le comportement rhéologique. En général, ils conduisent à un abaissement de la température de gélification ou de précipitation de la méthylcellulose ou de l'hydroxypropylcellulose. En effet, les méthylcelluloses ont la propriété originale de former des gels à température élevée. Du fait que la carboxyméthylcellulose est un polymère anionique, son comportement en solution en présence de sels est différent de celui des dérivés non ioniques.

Les dérivés cationiques, notamment la cellulose quaternisée ont été très peu étudiés. On peut cependant citer le document japonais JP-A-06114210 qui concerne la préparation de cellulose cationique par traitement de méthylcellulose en présence de métacrylate de diméthylaminoéthyle en milieu aqueux. Le composé obtenu est utilisé en floculation pour le traitement des eaux. Le document allemand DE-A-3644579 décrit la préparation d'une cellulose quaternisée de degré de substitution approximatif de 0,08 en mettant en contact la cellulose avec le chlorure de chloro-1-hydroxy-2-propyltriméthylammonium et dont la principale revendication est l'utilisation du produit dans le traitement des boues. Or, les facteurs qui limitent l'utilisation actuelle de la cellulose cationique comme d'autres dérivés de polymères végétaux dans la floculation sont le déficit de charge ionique et le déficit de masse.

Pour arriver à obtenir de la cellulose cationique de degré de substitution élevé, il est nécessaire d'utiliser un fort excès d'agents cationiques et des solvants relativement toxiques et polluants. Aujourd'hui ces méthodes restent valables, mais sur le plan économique sont d'un coût prohibitif.

On peut néanmoins éviter l'utilisation d'un fort excès de réactif cationique et améliorer le rendement de la réaction (en terme de degré de substitution) en ajoutant un cosolvant tel que l'isopropanol ou tout autre alcool encombré. Cet ajout de cosolvant permet à la fois de minimiser la dégradation du réactif cationique pendant la réaction et de mieux homogénéiser le milieu réactionnel favorisant ainsi un meilleur contact entre le substrat et le réactif.

Par ailleurs, la principale application de la cellulose cationique revendiquée dans les différents brevets se limite à la floculation. Il n'existe pas actuellement, à la connaissance de la Demanderesse, de la cellulose cationique sur le marché utilisée en cosmétique en tant qu'épaississant et/ou en tant que stabilisant d'émulsions ou toutes autres applications dans ce domaine.

Aussi un des buts de la présente invention est-il de fournir un procédé pour obtenir des microfibrilles de cellulose cationiques sous forme déshydratée, qui permet de pallier les inconvénients de l'art antérieur.

Un autre but de l'invention est de fournir un tel procédé qui permet d'obtenir des microfibrilles de cellulose cationiques trouvant des applications dans un grand nombre d'industries.

Ces buts ainsi que d'autres qui apparaîtront par la suite, sont atteints par un procédé pour obtenir des microfibrilles de cellulose cationiques, qui est, selon la présente invention, caractérisé par le fait que l'on met des fibres de cellulose en contact avec un agent cationique à une température comprise entre 20 et 90°C en présence d'un agent alcalin tel que la soude, et que l'on fait passer les fibres cellulosiques cationiques obtenues au moins une fois dans un homogénéisateur haute pression.

Avantageusement, l'agent cationique est un réactif époxy répondant à la formule (1) : 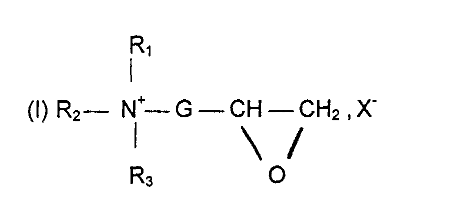 dans laquelle les substituants R1, R2, R3 sont, de préférence, identiques et choisis parmi les radicaux méthyle et éthyle, l'un d'eux pouvant être un atome d'hydrogène, X⁻ représentant Cl⁻, Br⁻, l⁻ et G étant un espaceur hydrocarboné comptant de 1 à 6 atomes de carbone.

Selon une variante de réalisation, l'agent cationique est un réactif de formule générale (II): 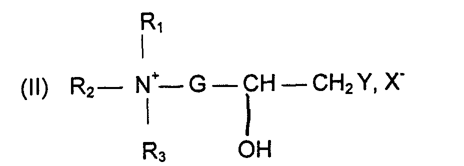 dans laquelle les substituants R₁, R₂, R₃ sont, de préférence, identiques et choisis parmi les radicaux méthyle et éthyle, l'un d'eux pouvant être un atome d'hydrogène, G étant un espaceur hydrocarboné comptant de 1 à 6 atomes de carbone, X⁻ représentant Cl⁻, Br⁻, I⁻ et Y représentant Cl, Br, ou I.

De préférence, la quantité d'agent alcalin mise en oeuvre est de 10⁻³ à 1, de préférence de 10⁻² à 10⁻¹ équivalent molaire par rapport au nombre de mole de l'anhydroglucose lorsque l'agent cationique est celui de la formule (I) ; cette quantité est augmentée, lorsque l'agent cationique est celui de la formule (II), d'une proportion correspondant à la neutralisation de l'agent cationique de formule (II).

Avantageusement, on met la cellulose cationique ainsi obtenue en suspension dans l'eau à une concentration comprise entre 0,5 et 10 %, de préférence de 1 à 5 % de matière sèche et l'on fait passer cette suspension au moins une fois dans un homogénéisateur haute pression.

Grâce à la cationisation, on peut obtenir deux types de dérivés de celluloses cationiques, selon le degré de substitution. Lorsque celui-ci est compris entre 0,1 et 0,7, ce sont les microfibrilles de cellulose en majorité insolubles dans l'eau qui, après passage dans l'homogénéisateur haute pression, deviennent un gel transparent. En revanche, lorsque le degré de substitution est supérieur ou égal à 0,7, le produit est hydrosoluble. Les dérivés cationiques possèdent une activité antimicrobienne quel que soit le degré de substitution.

Les microfibrilles de cellulose cationiques ainsi obtenues trouvent des applications dans plusieurs domaines techniques tels que :
- agent épaississant et agent stabilisateur dans le domaine des cosmétiques,
- agent épaississant, colloïde protecteur, et agent de suspension des pigments dans le domaine des peintures,
- agent filmogène de couchage, adhésif pour surfaçage et de rétention de charges en papeterie,
- agent épaississant, agent de suspension, liant, filmogène, stabilisateur pour émulsions dans les industries chimiques,
- agent floculant pour les bactéries et les levures notamment dans le traitement des eaux.

La présente invention a permis de découvrir d'une manière surprenante que la cellulose cationisée, passée dans un homogénéisateur haute pression présente des propriétés rhéologiques très intéressantes (viscosité élevée à une faible concentration, en écoulement à caractère non newtonien des suspensions, etc.). De plus, elle peut être déshydratée tout en préservant le comportement rhéologique à la redispersion. En effet, on observe une réversibilité de la déshydratation-réhydratation des fibres. Cette réversibilité est confirmée par une étude comparative du comportement rhéologique des deux suspensions à même concentration (l'une obtenue à partir du gel hydraté, l'autre à partir du gel séché). Ce résultat est rendu possible par les effets conjugués de la cationisation et de l'individualisation des fibres par le passage dans un homogénéisateur haute pression.

La combinaison de la cationisation apportée par la modification chimique et de l'individualisation des fibres réalisée par passages répétés dans un homogénéisateur à haute pression a permis également de donner au produit un aspect « gel transparent » à partir d'un degré de substitution moyen donné (DS>0,1). En effet, le procédé de préparation a permis d'obtenir deux types de produits dont les caractéristiques diffèrent selon leur degré de substitution. Si le degré de susbstitution moyen est compris entre 0,1 et 0,7, le produit est majoritairement insoluble et forme, après passage dans l'homogénéisateur à haute pression, un gel transparent de viscosité élevée entre 1 et 3 % de matière sèche, et lorsque ce degré de substitution est supérieur ou égal à 0,7, le composé obtenu est hydrosoluble.

On entend par gel transparent, un système hétérogène dans lequel une phase insoluble très gonflée est dispersée dans une solution aqueuse contenant une faible fraction du polymère de DS supérieur hydrosoluble ou tout simplement un système homogène ou non dans lequel des microfibrilles de cellulose cationiques dispersées dans l'eau entre 0,5 et 5 % de matière sèche, de préférence entre 1 et 3 % de matière sèche donnent une viscosité élevée et présentent un aspect transparent.

Conformément à l'invention, le gel transparent obtenu peut être ensuite séché sous forme de poudre jusqu'à une matière sèche de 100 %. En réhydratant cette poudre, on retrouve intégralement les propriétés physico-chimiques initiales du gel.

Parmi ces dérivés, on préfère pour leurs bonnes propriétés rhéologiques et leur aspect « gel transparent », recherchés dans plusieurs domaines d'applications, notamment la cosmétique, les composés ayant des DS compris entre 0,1 et 0,7. L'évaluation est réalisée par une étude du taux de gonflement et de la rhéologie du produit. Les microfibrilles cationisées en suspension dans l'eau présentent une reprise en eau très élevée qui peut être de 60 g/g de substance sèche pour un DS supérieur à 0,2. Ce gonflement reste élevé même dans un milieu à forte concentration en sel (à une concentration en NaCI de 1 M ce gonflement est de 35 g/g sec). Ce gonflement est réversible après séchage. Le gonflement est peu sensible à la présence de solvant organique polaire (pour un mélange EtOH/eau en proportion volumique de 50/50 ce gonflement est de 48 g/g).

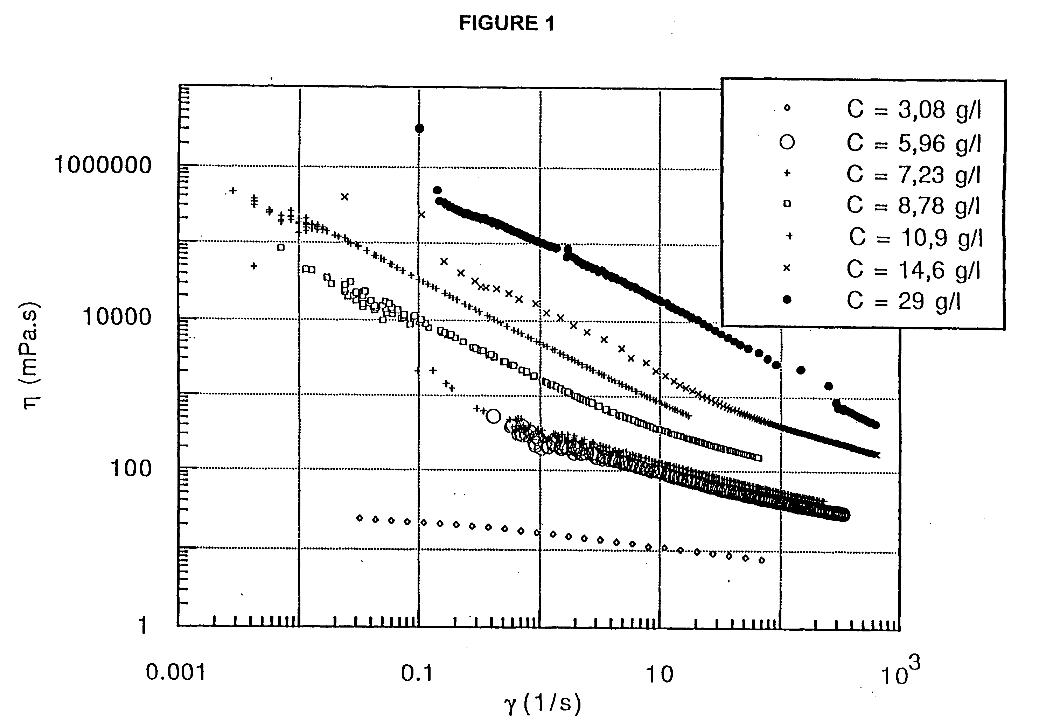

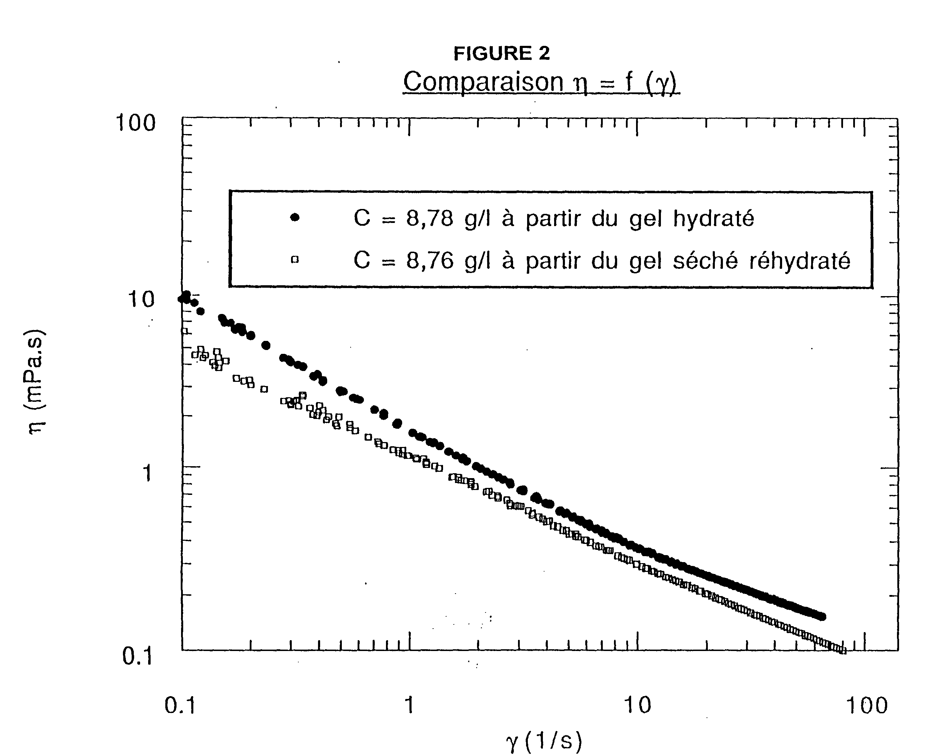

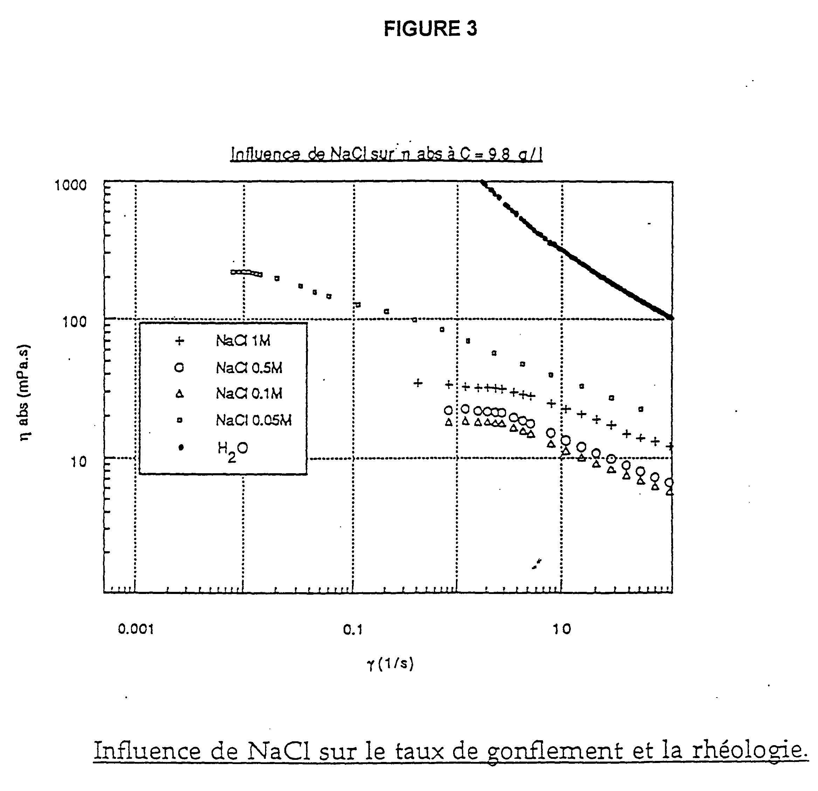

L'étude rhéologique montre qu'en écoulement les suspensions présentent un caractère non newtonien et des viscosités dépendant fortement de la concentration (Figure 1). Le comportement rhéologique est réversible : on retrouve pratiquement la même rhéologie après séchage et réhydratation du gel (Figure 2). La viscosité des suspensions est sensible à la présence de sels en particulier à faible gradient de vitesse (Figure 3).

Comme le montrent les résultats regroupés dans le tableau I ci-après, de façon originale la viscosité des suspensions augmente avec la température alors que pour un épaississant polymère tel que le xanthane, elle décroit :

**Tableau I**

| **Echantillon** | **Viscosité** | | | |
|---|---|---|---|---|
| | **à 20°C** **mPa.s** | **à 35°C** **mPa.s** | **à 50°C** **mPa.s** | **à 60°C** **mPa.s** |
| Xanthane (c=2 g/l) | 490 | 400 | 270 | |
| Microfibrilles cationisées DS=0,60 (c=15 g/l) | 740 | 880 | 980 | 1100 |
| Microfilbrilles cationisées DS=0,6 (c=21,6 g/l) | 3500 | 5000 | 6000 | |
| Gradient de vitesse égal à 1s⁻¹. | | | | |

Cette propriété peut avantageusement être utilisée dans le domaine de la fabrication des boues de forage ou à la récupération assistée du pétrole.

On met en évidence l'existence d'un seuil d'écoulement pour une concentration en fibres de l'ordre de 10 g/l et plus. Les suspensions dans ce domaine de concentration présentent une faible thixotropie qui augmente quand la concentration augmente.

En dynamique, le comportement d'une suspension de microfibrilles cationiques devient typique d'un gel (G'>G") au-delà d'une concentration en microfibrilles de 10 g/l dans l'eau. Le caractère de gel augmente lorsque la température augmente.

Les fibres cationisées préparées peuvent être utilisées comme floculant d'une suspension de levure. L'efficacité dépend du DS et de la concentration en produit ajouté et est meilleure que pour différents polymères utilisés à cet effet. La floculation est rapide et permet la séparation des levures par filtration, centrifugation ou décantation. Le procédé est utilisable à de nombreuses suspensions de particules divisées (en particulier quand leur charge superficielle est négative).

L'ensemble de ces propriétés (taux de gonflement élevé, comportement rhéologique réversible après séchage et réhydratation, viscosité augmentant avec la température, etc.) font que les microfibrilles de cellulose cationiques peuvent être utilisées en cosmétique (en tant qu'épaississant, stabilisant d'émulsion). On préférera les microfibrilles cationiques de DS 0,2 à 0,7, particulièrement de DS 0,3 à 0,6, plus particulièrement de DS 0,5-0,6.

L'invention a également pour objet des compositions cosmétologiques caractérisées en ce qu'il consiste à incorporer de 0,1 à 50 % et de préférence de 1 à 10 % en poids d'un dérivé ou d'un mélange de dérivés de degrés de substitution différents suivant l'invention.

Le procédé conforme à l'invention consiste en premier lieu à faire réagir de la cellulose avec un réactif cationique amino-tertiaire, ammonium quaternaire, phosphonium quaternaire ou sulfonium tertiaire et est caractérisé par le fait que la réaction est mise en oeuvre à une température de 20 à 90°C, de préférence de 40 à 80°C, en présence d'un agent alcalin, notamment la soude, et avec comme solvant ou cosolvant de la réaction de 10 à 100 % d'eau, ou d'alcools de préférence l'isopropanol, le tertio-butanol, le 2-éthyl 1-héxanol ou l'éthanol par rapport à la matière sèche de la cellulose, ou de mélange d'eau et d'alcool.

De préférence, le mélange réactionnel est une dispersion de fibres dans un mélange eau-alcool encombré, de préférence une dispersion eau-isopropanol. Le milieu réactionnel est refroidi en fin de réaction, éventuellement neutralisé par un acide, filtré et séché. On peut également, en fin de réaction, pour faciliter la filtration et le séchage, ajouter dans le milieu réactionnel, un alcool ou tout autre solvant pouvant déshydrater les fibres, de préférence de l'éthanol, de l'isopropanol, du méthanol etc. Le composé résultant est dérivé de la cellulose.

Suivant un mode de réalisation avantageux, le procédé de la présente invention est mis en oeuvre de 10⁻³ à 1, et de préférence de 10⁻² à 10⁻¹, équivalent molaire d'un catalyseur basique tels que des hydroxydes et des carbonates de métaux alcalins comprenant les hydroxydes et carbonates de sodium, de potassium et de lithium, les amines comprenant les amines hydrocarbonées aliphatiques comptant 12 atomes de carbone au moins, ou plus particulièrement les amines tertiaires telles que la pyridine. La base est destinée à donner un milieu réactionnel alcalin ; le pH doit être d'au moins 9,5 environ pendant toute la réaction.

Suivant un autre mode de réalisation avantageux du procédé, la quantité de réactif cationique mise en oeuvre est de 0,05 à 50 équivalents molaires, de préférence 0,1 à 10 équivalents molaires par équivalent molaire de motifs anhydroglucose de la cellulose. Un éther de cellulose cationique particulièrement préféré présente un DS de 0,1 à 0,6 environ.

Suivant un autre mode de réalisation avantageux du procédé, le réactif cationique est de forme époxy (formule 1) ou répondant à la formule (II). 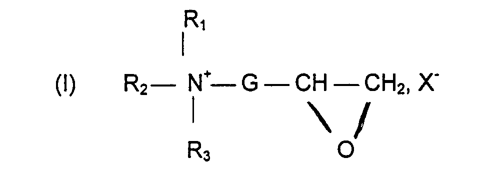 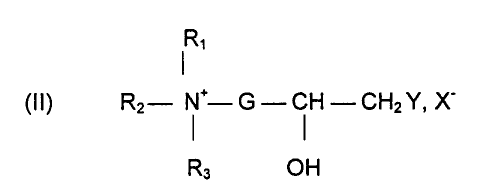 correspondant à des dérivés tertiaires ou quaternaires suivant que l'un des substituants de l'atome d'azote est un hydrogène ou non, les substituants dans la mesure où ils ne sont pas un atome d'hydrogène, étant de préférence identiques et choisis parmi les radicaux méthyle ou éthyle, Y étant un halogène Cl, Br, ou I et X⁻ étant un halogénure Cl⁻, Br⁻, I⁻, G étant un espaceur à squelette hydrocarboné comptant de 1 à 6 atomes de carbone. Les exemples de réactifs cationiques comprennent les produits réactionnels de l'épichlorhydrine et de la triméthylamine, la N,N-diméthyl-N-éthylamine, la N-méthyl-N,N-diéthylamine, la diméthylamine, la tri-n-butylamine, la N,N-diméthyl-N-butylamine, la N,N-diméthyl-N-phénylamine, la pipéridine, la N-méthylpipéridine.

Les réactifs de formule (I) sont très réactifs et en présence d'ions alcalins OH⁻, réagissent avec les groupements hydroxyles de la cellulose. Les réactifs de formule (II), sous l'action des ions OH⁻ se transforment d'abord en forme glycidyle (formule I) avant la condensation sur la cellulose. Il faudra donc tenir compte de l'alcalinité consommée pour cette conversion dans l'ajustement du taux de catalyseur.

Des exemples avantageux de réactifs dérivés d'amine tertiaires sont :
- le chlorhydrate de diéthylamino-2,3 époxy propane de formule : 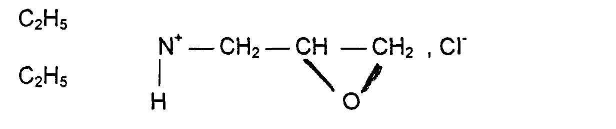 désigné par DEAEP
- le chlorure de diéthylamino-hydroxy-2, chloro 3-propane de formule : 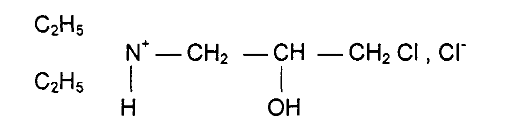 désigné par DEACP

Des exemples avantageux de réactifs quaternaires sont :
- le chlorure de 2,3-époxy-propyl-triméthylammonium de formule : 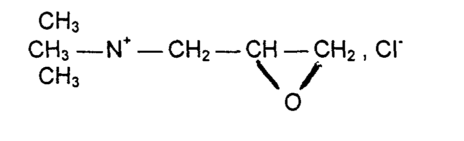 désigné par TMACP
- le chlorure de 3-chloro, 2-hydroxypropyltriméthylammonium 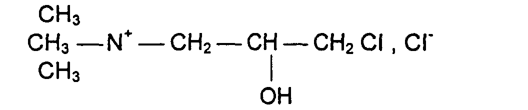 désigné par TMACP

En travaillant dans le domaine préférentiel de 40 à 60°C, la réaction peut être effectuée pendant une durée allant de 0,5 heures à 24 heures, de préférence entre 5 et 12 heures.

Pour préparer les éthers de cellulose avec un degré de substitution relativement élevé selon la présente invention, la matière sèche de la cellulose de départ est particulièrement importante. La matière sèche peut être comprise entre 2 à 80 %, de préférence entre 20 et 70 %. Une plus forte teneur en eau donne un produit de très faible DS et une plus faible teneur en eau et en l'absence de tout autre solvant ou cosolvant rend difficile l'homogénéisation du milieu réactionnel et donne également un mauvais rendement en terme de DS.

En fin de réaction, le milieu réactionnel peut être évidemment neutralisé par un quelconque des acides minéraux courants tels que l'acide sulfurique, l'acide chlorhydrique ou l'acide phosphorique ou bien un acide organique, par exemple l'un des acides carboxyliques aliphatiques de 1 à 6 atomes de carbone, de préférence l'acide acétique. Après neutralisation, le produit en suspension peut être récupéré par simple filtration, centrifugation ou tout autre système pouvant séparer le solide du liquide et est ensuite séché selon la matière sèche désirée. De plus, pour faciliter la filtration et le séchage du produit on peut ajouter au milieu réactionnel un alcool ou tout autre solvant pouvant précipiter les microfibrilles, ou le traiter à l'eau froide. Ainsi, le milieu réactionnel est ramené à température ambiante et on y ajoute 1 à 10 volumes d'eau froide (température comprise entre 0 et 5°C environ), de préférence 1 à 5 volumes d'eau froide par rapport au volume du milieu réactionnel. On laisse agiter le mélange pendant environ 20 minutes afin de mieux précipiter les fibres, ensuite on récupère le produit par simple filtration.

Selon une autre forme de réalisation, la réaction de cationisation peut être effectuée sous atmosphère d'azote, et à une température comprise entre 20 et 90°C, de préférence entre 20 et 50°C, par condensation de l'épichlorhydrine et d'une amine tertiaire en présence de la cellulose et de la soude comme catalyseur. Les amines tertiaires qui réagissent le mieux sont celles qui contiennent au moins deux groupements méthyle liés à l'azote. L'encombrement stérique est alors minimum, d'où un contact plus facile entre l'épichlorhydrine et le doublet libre de l'amine tertiaire. Toutefois, la triéthylamine réagit aussi très bien.

Suivant un autre mode de réalisation avantageux du procédé, la quantité de l'épichlorhydrine et de l'amine tertiaire mise en oeuvre est de 0,05 à 50 équivalents molaires, de préférence 0,1 à 10 équivalents par équivalent molaire de motifs d'anhydroglucose de cellulose.

Selon une variante de réalisation, la cationisation est effectuée sur la cellulose préalablement passée dans un homogénéisateur haute pression. Dans ce cas, la cellulose est mise en suspension entre 0,5 et 10 % de matière sèche, de préférence entre 1 et 5 % de matière sèche pour être ensuite traitée à travers un appareil de type Nanojet (société SODEXIM), ou un microfluidiseur (Microfluidics International Corporation) ou encore un appareil de type Manton-Gaulin ou tout autre type d'appareil pouvant émulsifier, homogénéiser, individualiser les microfibrilles de cellulose ou disperser des solutions liquide-liquide, ou solide-liquide. Cette suspension subit alors des pressions pouvant aller jusqu'à 15.10⁷ Pa (1500 bars). Le passage à travers l'homogénéisateur haute pression donne naissance à des forces de cisaillement, des turbulences et des phénomènes de cavitation. La conjugaison de ces forces entraîne l'individualisation des microfibrilles de cellulose.

En second lieu, la cellulose cationique obtenue précédemment est mise en suspension entre 0,5 et 10 % de matière sèche, de préférence entre 1 et 5 % de matière sèche pour être ensuite traitée à travers un appareil de type Nanojet (société SODEXIM), ou un microfluidiseur (Microfluidics International Corporation) ou encore un appareil de type Manton-Gaulin ou tout autre type d'appareil pouvant émulsifier, homogénéiser, individualiser les microfibrilles de cellulose ou disperser des solutions liquide-liquide, ou solide-liquide. Cette suspension subit alors des pressions pouvant aller jusqu'à 15.10⁷ Pa (1500 bars). L'impact donne naissance à des forces de cisaillement, des turbulences et des phénomènes de cavitation. La conjugaison de ces forces entraîne l'individualisation des microfibrilles de cellulose.

On peut passer une à plusieurs fois, de préférence une à 10 fois, la suspension de cellulose cationique pour obtenir une suspension stable de microfibrilles de cellulose cationiques individualisées.

On obtient alors, après cette opération, des microfibrilles de cellulose cationiques sous forme de gel transparent ayant une matière sèche comprise entre 0,5 et 10 %, de préférence comprise entre 1 et 5 %. Les microfibrilles de cellulose cationiques sont obtenues sous forme de gel transparent possédant de très hautes viscosités.

A l'issue, les microfibrilles de cellulose cationiques peuvent être également mises sous forme de poudre ayant une matière sèche pouvant aller jusquà 100%.

Le mode de séchage peut être réalisé en ajoutant à une suspension aqueuse de microfibrilles cationiques sous forme de gel transparent (3 % de matière sèche) de l'isopropanol ou de l'éthanol ou tout autre solvant pouvant déshydrater les fibres. Les microfibrilles de cellulose cationiques déshydratées sont récupérées par filtration sur büchner ou fritté sous pression réduite. Le produit obtenu peut être séché à l'étuve sous vide à une température comprise entre 30 et 80°C pendant 8 à 24 heures.

On peut également sécher cette suspension aqueuse de microfibrilles de cellulose cationiques par microonde ou par tout autre mode de séchage adapté.

Cette poudre de microfibrilles de cellulose une fois réhydratée, retrouve ses propriétés rhéologiques (sa viscosité initiale et son aspect de gel transparent, etc.). La poudre est mise en suspension aqueuse entre 0,5 et 10 % de matière sèche, de préférence entre 1 et 5 % de matière sèche.

L'invention concerne également des dérivés de microfibrilles de cellulose cationiques de DS compris entre 0,1 et 1 sans utilisation d'un fort excès de réactifs cationiques selon le DS souhaité, pouvant être déshydratés ou séchés sans modifier les propriétés rhéologiques à la réhydratation, qui s'avèrent doués de propriétés, notamment épaississantes et stabilisantes d'émulsions, supérieures à celles des meilleurs épaississants utilisés jusqu'ici, et floculantes dans le traitement des eaux. Ces dérivés de microfibrilles de cellulose s'avèrent également posséder de bons pouvoirs de rétention d'eau, de fixation pour fongicide, d'être de bons agents de cohésion pour formulations à base de ciment et de plâtre et de bons agents filmogènes, de couchage et de suspension des pigments pour formulations des peintures à émulsions.

Les microfibrilles de cellulose cationiques présentent en effet un ensemble de propriétés intéressantes. Elles peuvent remplir, outre les propriétés citées précédemment, le rôle de colloïde protecteur, de liant. Leur caractère épaississant peut être recherché en s'appuyant sur leur remarquable stabilité.

Elles présentent également des propriétés antibactériennes qui sont recherchées dans de nombreuses applications.

On peut envisager l'utilisation des produits de l'invention dans le domaine de l'agriculture comme agent de fixation pour pesticides, dans le bâtiment en tant qu'agent rétenteur d'eau ou agent de cohésion pour les formulations à base de ciment et de plâtre ou en tant qu'agent de modification de la rhéologie et de rétention d'eau. Ils peuvent être utilisés dans le domaine cosmétique en tant qu'épaississant et stabilisant (ils améliorent l'onctuosité, stabilisent les mousses, accroissent la compatibilité des tensioactifs dans les lotions, les crèmes, les produits capillaires et shampooings). On peut également citer l'industrie des peintures, vernis et encres, du papier et des traitements des eaux et des adjuvants pour boue de forage ou de récupération assistée du pétrole.

La proportion de microfibrilles de cellulose cationiques conformes à l'invention devant être mis en oeuvre dans les applications principales varie en fonction de l'industrie concernée ; on trouve à ce propos des indications numériques dans les exemples.

Parmi ces dérivés, on préfère pour leur bon pouvoir épaississant, leur viscosité très élevée et leur aspect « gel transparent », les composés ayant de degré de substitution entre 0,2 et 0,7.

D'une façon générale, les microfibrilles de cellulose cationiques obtenues conformément à l'invention peuvent présenter des degrés de substitution variables, fonction de la quantité de réactif mis en jeu, du temps et de la température de réaction.

Le degré de substitution est calculé en fonction du taux d'azote déterminé par la méthode Kjeldahl. Il est également calculé par dosage des chlorures (lorsque le produit est isolé sous forme de chlorure) et par microanalyse du carbone, de l'azote et du chlore.

Le rendement de la réaction de cationisation est, dans le procédé conforme à l'invention, voisin de 100 %. Ce rendement est déterminé par le dosage de l'azote fixé.

Pour illustrer ce qui précède, on indique ci-après un certain nombre d'exemples de préparation de microfibrilles de cellulose cationiques conformes à l'invention sans toutefois présenter un caractère limitatif.

Ces exemples permettront à l'homme du métier de mieux comprendre la mise en oeuvre de l'invention ainsi que les avantages de celle-ci.

### Exemple 1

On prépare une suspension de son à 12,5 % de matière sèche (100 g de matière sèche pour 700 g d'eau osmosée dans une solution de soude 1 molaire (soit 28 g de NaOH pastille).

Le milieu réactionnel est homogénéisé dans un réacteur agité, puis porté à une température de 80°C pendant une heure.

Après la cuisson, le mélange est dilué au 1/3 puis acidifié avec 6,7 g d'acide sulfurique (H₂SO₄ à 96 %).

Le pH du mélange est alors de 9,5. Le mélange est centrifugé sur une centrifugeuse laboratoire à 4080 g pour séparer les boues du jus.

Les boues sont reprises puis légèrement diluées de manière à obtenir une matière sèche de 10 % (ajout de 34 g H₂O). On chauffe la solution à 80°C puis on met en contact du peroxyde d'hydrogène à 80 % à raison de 35 % par rapport à la matière sèche pendant 1 h 15. Le mélange est centrifugé à 4080 g pendant 5 minutes, on récupère alors les boues qui sont lavées avec de l'eau et à nouveau centrifugées. On récupère alors 229,3 g de cellulose purifiée de son.

A 62,4 g (410 mmoles) de chlorure de 2,3-époxy-propyltriméthylammonium, on ajoute 20 g (124,1 mmoles) de cellulose purifiée de son et 1,6 g (40 mmoles) de soude préalablement dissous dans 1,6 g d'eau. On agite vigoureusement le mélange et on ajoute ensuite 60 g d'eau. On porte puis on maintient le milieu réactionnel à 50°C pendant 12 heures. En fin de réaction, la suspension est filtrée sur büchner ou sur fritté sous pression réduite. Le gâteau est lavé 2 fois avec de l'éthanol 95 %, puis séché à l'étude sous vide une nuit. On récupère un produit sous forme de poudre de DS 0,60.

On prépare 500 ml d'une suspension aqueuse de la cellulose cationique obtenue à 3 % de matière sèche. Cette suspension est ensuite passée 1 fois dans l'homogénéisateur haute pression sous une pression de 8.10⁷ Pa (800 bars). L'opération est réalisée à température ambiante. Après ce traitement, le produit se présente sous forme d'un gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemple 2

A un mélange de 20 g (124,1 mmoles/anhydroglucose) de cellulose purifiée de son de l'exemple 1 mis en suspension dans 80 g d'eau et de 31,2 g (205,8 mmoles) de chlorure de 2,3-époxy-propyltriméthylammonium, on ajoute 1,6 g (40 mmoles) de soude dissous dans 1,6 g d'eau. Ce mélange est agité vigoureusement à l'aide d'un agitateur mécanique. On porte le milieu réactionnel (un milieu réactionnel hétérogène) à 50°C pendant 5 heures. En fin de réaction, le milieu réactionnel est neutralisé par 2,40 g (30 mmoles) d'acide acétique. On filtre la suspension sur un büchner ou un fritté de porosité 3. Le gâteau obtenu est séché à l'étude sous vide pendant 12 heures. Le degré de substitution est calculé en fonction du taux d'azote fixé. La détermination de taux d'azote est faite par la méthode Kjeldahl. Le produit final présente un degré de substitution de 0,24.

### Exemple 3

Un autre essai est réalisé d'une manière identique, sauf qu'en fin de réaction, le milieu réactionnel n'est pas neutralisé. On filtre la suspension sur un büchner ou un fritté de porosité 3 sous pression réduite. Le gâteau est lavé deux fois avec 100 ml d'éthanol 95 %. Le produit récupéré est laissé sécher sous vide à l'étuve pendant 12 heures. La cellulose cationique obtenu a un degré de substitution de 0,24.

### Exemple 4

On prépare 500 ml d'une suspension aqueuse de la cellulose cationique obtenue dans l'exemple 2 ou 3 à 3 % de matière sèche. Cette suspension est ensuite passée 1 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre.

Cette poudre, réhydratée à 3 % de matière sèche, se remet sous son aspect de gel transparent et conserve ses propriétés rhéologiques initiales avant séchage.

### Exemple 5

A 62,4 g (410 mmoles) de chlorure de 2,3-époxy-propyltriméthylammonium, on ajoute 20 g (124,1 mmoles) de cellulose purifiée de son et 1,6 g (40 mmoles) de soude préalablement dissous dans 1,6 g d'eau. On agite vigoureusement le mélange et on ajoute ensuite 60 g d'eau. On porte puis on maintient le milieu réactionnel à 50°C pendant 12 heures. En fin de réaction, la suspension est filtrée sur büchner ou sur fritté sous pression réduite. Le gâteau est lavé 2 fois avec de l'éthanol à 95 %, puis séché à l'étuve sous vide une nuit. On récupère un produit sous forme de poudre de DS 0,60.

On prépare 500 ml d'une suspension aqueuse de cellulose cationique obtenue à 3 % de matière sèche. Cette suspension est ensuite passée 1 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemple 6

L'essai est réalisé d'une manière identique que l'exemple 5, sauf qu'en fin de réaction, avant de filtrer la suspension, on ajoute d'abord de l'éthanol dans le milieu réactionnel afin de faciliter la récupération du produit. La suite du traitement est identique que l'exemple 3. Le degré de substitution obtenu est de 0,6 (taux d'azote fixé : 3,34 % en masse).

### Exemple 7

15 g de cette cellulose cationique obtenue selon l'exemple 6 sont mis en suspension à 3 % de matière sèche dans l'eau. La suspension de cellulose cationique est pompée à travers une chambre de pression d'un homogénéisateur haute pression. Dans la chambre de pression, le produit subit une pression de 10⁸ Pa (1000 bars). Des forces de cisaillement et de cavitation énormes entrainent une réorganisation des microfibrilles de cellulose cationiques sous forme de gel transparent de très haute viscosité. L'opération est réalisée à température ambiante. Le produit est passé 1 fois à travers la chambre de pression et on constate une importante augmentation de sa viscosité. La suspension de microfibrilles de cellulose cationiques sous forme de gel transparent est précipitée dans 500 ml d'isopropanol. Le précipité est récupéré par filtration sur büchner et séché à l'étuve sous pression réduite à 40°C pendant 12 heures. Le produit séché est réhydraté afin d'étudier sa rhéologie. Les études ont montré que les propriétés physico-chimiques initiales sont conservées.

Les microfibrilles de cellulose obtenues à l'issue des exemples 1 à 6 ont un DS de 0,6 et se présentant sous forme de gel transparent à 3 % de matière sèche dans l'eau : ce gel peut être considéré comme un système hétérogène dans lequel une phase insoluble très gonflée est dispersée dans une solution aqueuse d'une faible fraction constituée de polymères hydrosolubles (de l'ordre de 15 % pour un DS de 0,6).

Après séchage, le produit se redisperse immédiatement dans l'eau. Le taux de gonflement est recalculé pour une concentration en matière sèche d'environ 7 g/l (à partir d'un gel séché). On obtient une valeur de 56,2 g H₂O / g sec. Pour une concentration de 7,23 g/l à partir du gel hydraté, on obtient une valeur de 57,85 g H₂O/g sec.

### Exemple 8

Dans un réacteur muni d'un système d'agitation mécanique, d'une double enveloppe, on introduit 20 g de cellulose à 50 % de matière sèche (10 g de cellulose sèche, 65 mmoles). On ajoute ensuite 31,2 g (205,8 mmoles) de chlorure de 2,3-époxy-propyltriméthlammonium. On laisse bien homogénéiser le mélange réactionnel et on additionne ensuite 3,2 g d'une solution aqueuse de soude à 50 %. On laisse réagir pendant 2 heures à 50°C. Au bout de 2 heures, le milieu réactionnel s'épaissit (au fur et à mesure que la réaction avance le milieu réactionnel devient de plus en plus visqueux). On additionne alors 50 à 100 ml d'eau jusqu'à obtenir à nouveau un milieu réactionnel beaucoup moins visqueux. On laisse la réaction se poursuivre pendant 10 heures à 50°C. Le mélange réactionnel est traité en filtrant le produit sous vide. Le produit est ensuite lavé 2 fois avec 50 ml d'éthanol 95 %. Le résidu obtenu est séché à l'étuve sous vide une nuit. Le taux d'azote fixé déterminé par la méthode Kjeldahl est de 2,46 % sur sec ce qui correspond à un degré de substitution de 0,39.

On prépare 500 ml d'une suspension aqueuse de cellulose cationique à 3 % de matière sèche. Cette suspension est ensuite passée 3 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemple 9

A un mélange de 28,6 g de cellulose à 70 % de matière sèche (20 g : 124,1 mmoles) et 62,4 g (411,6 mmoles) de chlorure de 2,3-époxy-propyltriméthylammonium sont ajoutés 3,2 g d'une solution aqueuse de soude à 50 % en poids. On laisse agiter vigoureusement le mélange réactionnel à 50°C pendant environ 2 heures. Le milieu réactionnel devient visqueux, et on ajoute alors 50 à 100 ml d'eau. On laisse la réaction se poursuivre pendant 10 heures et le traitement est réalisé de la même manière que dans l'exemple 5. Le taux d'azote mesuré par la méthode Kejldahl est de 2,27 % sur sec, ce qui correspond à un DS de 0,35.

On prépare 500 ml d'une suspension aqueuse à 3 % de matière sèche du produit obtenu. Cette suspension est ensuite passée 3 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemple 10

A partir d'une cellulose purifiée de son, on réalise l'individualisation des microfibrilles de cellulose sur 1 I d'une suspension de cellulose à 2 % de matière sèche.

Le procédé est le suivant :
1 l d'une suspension de cellulose purifiée à 2 % de matière sèche obtenue après délignification et blanchiment du résidu ligno-cellulosique issu du son selon le procédé de la présente invention, est pompée à travers une chambre de pression. Dans cette chambre de pression, le produit subit une pression de 10⁸ Pa (1000 bars). Des forces de cisaillement et de cavitation énormes entraînent une réorganisation des microfibrilles de cellulose sous forme d'un gel d'une très haute viscosité. L'opération est réalisée à température ambiante. Le produit est passé 5 fois à travers la chambre de pression, ce qui conduit à une augmentation importante de la viscosité. La suspension de microfibrilles de cellulose à 2 % de matière sèche obtenue présente alors une viscosité de 20.000 centipoises (viscosité mesurée au viscosimètre Brookfield DV II à 20°C avec un mobile 4 et à une vitesse de 3 rpm).

Afin d'effectuer la cationisation, on ramène la matière sèche de la suspension des microfibrilles de cellulose à 31,83 %.

A un mélange de 62,83 g de microfibrilles de cellulose à 31,83 % de matière sèche (20 g de microfibrilles sèches) et 96 g de chlorure de 2,3 époxypropyltriméthylammonium à 65 % de matière active soit 411 mmoles sont ajoutés 2,4 g d'une solution de soude à 50 % en poids. On homogénéise le mélange pendant quelques minutes et on ajoute 60 ml d'eau. La réaction est réalisée à 50°C pendant 12 heures en agitant vigoureusement. En fin de réaction, le produit est filtré sous pression réduite et lavé 3 fois avec 150 ml d'éthanol à 95 %. Le produit est séché à l'étuve sous vide et se présente sous forme de poudre très fine. Le taux d'azote fixé est de 3,72 % (déterminé par la méthode Kjeldahl) qui correspond à un degré de substitution de 0,72.

15 g de ce produit sont mis en suspension à 3 % de matière sèche dans l'eau. Cette suspension de cellulose cationique est de nouveau pompée à travers une chambre de pression d'un homogénéisateur haute pression. Dans la chambre de pression, le produit subit une pression de 10⁸ Pa (1000 bars). Des forces de cisaillement et de cavitation énormes entraînent une réorganisation des microfibrilles de cellulose cationiques sous forme de gel transparent de très hautes viscosités. L'opération est réalisée à température ambiante. Le produit est passé 1 fois à travers la chambre de pression, ce qui conduit à une augmentation importante de la viscosité. La suspension de microfibrilles de cellulose cationiques sous forme de gel transparent est précipitée dans 500 ml d'isopropanol. Le précipité est récupéré par filtration sur büchner et séché à l'étuve sous pression réduite à 40°C pendant 12 heures. Le produit séché est réhydraté afin d'étudier sa rhéologie. Les études ont montré que les propriétés physico-chimiques initiales sont conservées.

### Exemple 11

A 20 g de cellulose à 50 % de matière sèche (10 g de cellulose sèche 65 mmoles), on ajoute 9,85 g (65 mmoles) de chlorure de 2,3 époxy-propyltriméthylammonium et 100 ml de 2-propanol. On mélange le milieu réactionnel en agitant vigoureusement. On ajoute ensuite 1,6 g d'une solution de soude à 50 % en poids. On porte le tout à 50°C et on laisse agir pendant 12 heures. On filtre ensuite le produit et on lave 2 fois avec 50 ml d'éthanol à 96 %. Le produit récupéré est séché à l'étuve sous vide à 40°C pendant une nuit. Le taux d'azote fixé donné par le dosage Kjeldahl est 1,96 % (DS de 0,29).

On prépare 500 ml d'une suspension aqueuse à 3 % de matière sèche du produit. Cette suspension est ensuite passée 3 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemple 12

A 32,7 g de cellulose purifiée de son à 61,18 % de matière sèche (20 g de cellulose sèche 130 mmoles), on ajoute 18,18 g (130 mmoles) de chlorure de 2,3-époxy-propyltriméthylammonium et 100 ml de tertiobutanol. On mélange le milieu réactionnel en agitant vigoureusement. On ajoute ensuite 1,6 d'une solution de soude à 50 %. On porte le tout à 50°C et on laisse réagir pendant 12 heures. On filtre ensuite le produit et on lave 2 fois avec 50 ml d'éthanol à 96 %. Le produit récupéré est séché à l'étuve sous vide pendant une nuit à 40°C. Le taux d'azote fixé donné par le dosage Kjeldahl est 1,70 % (DS de 0,24).

On prépare 500 ml d'une suspension aqueuse de cellulose cationique obtenue dans l'exemple 1 ou 2 à 3 % de matière sèche. Cette suspension est ensuite passée 3 fois dans l'homogénéisateur haute pression sous une pression de 8.10⁷ Pa (800 bars). L'opération est réalisée à température ambiante. Après ce traitement, le produit se présente sous forme de gel transparent possédant une très haute viscosité. Le gel est séché par microonde et mis sous forme de poudre. Cette poudre, réhydratée à 3 % de matière sèche, se remet sous son aspect de gel transparent et conserve ses propriétés rhéologiques initiales avant séchage.

### Exemples 13 à 17

La réaction se déroule sous atmosphère d'azote et à température ambiante. On introduit l'épichlorhydrine, la triéthylamine et 3.10⁻³ moles de cellulose. On ajoute de l'eau afin d'homogénéiser la suspension (2 à 10 ml). Après agitation, la soude est ajoutée et la réaction se poursuit pendant une durée variable. Le produit obtenu est lavé avec de l'eau et de l'éthanol. Les conditions expérimentales exactes et les caractéristiques des produits obtenus sont reportées dans le tableau Il ci-après.

### Exemples comparatifs 17 et 18

Comparaison du pouvoir stabilisant et épaississant de microfibrilles cationiques de DS 0,6 et des produits commerciaux.

Les émulsions sont réalisées avec le même taux d'épaississant. Les tests sont effectués avec et sans émulsifiant. L'émulsifiant utilisé est un tensioactif dérivé de blé décrit dans le brevet EP 0 699 472.

On prépare une solution à 2 % d'épaississant (sauf le carbopol 0,25 %), 15 % d'huile de paraffine ou de tournesol et 1 % de tensioactif (pour les tests effectués avec émulsifiant) et complétée avec de l'eau déminéralisée. On porte le mélange à 70°C et on homogénéise 30 secondes à 1500 rpm au polytron. On refroidit le mélange et on fait le test de vieillissement à 45°C pendant au moins 100 jours.

Les résultats du test de vieillissement sont consignés dans le tableau III suivant :
Résultats enregistrés au delà de 100 jours à 45°C
(stable +, non stable -)

**Tableau III**

| | **Exemple 17** | **Exemple 18** |
|---|---|---|
| **Références** | **Avec 1 % tensioactif** | **0 % tensioactif** |
| Amigel | + | + |
| Rhodicare T | + | + |
| Méthocel 40-202E | - | - |
| Méthocel J75MS | - | - |
| Carbopol Ultrez | + | + |
| Seppigel | + | + |
| Satialginate M280 | - | - |
| Microfibrilles cationiques | + | + |
| Témoin sans épaississant | - | - |

Les microfibrilles de cellulose, objet de la présente invention, sont comparées avec les épaississants commerciaux suivants :
Amigel est un polysaccharide commercialisé par Alban Muller
Rhodicare T est du Xanthane de Rhone-Poulenc

Les Methocels sont des méthylcelluloses commercialisées par Dow Chemical sous cette marque
Seppigel est un polyacrylate de Seppic
Satialginate M280 est un alginate de sodium commercialisé par Sanofi.

### Exemples 19 et 20

| Formulation shampooing | | |
|---|---|---|
| Tensioactif de l'exemple 6 du Brevet EP 0669472 | 10 g | 15 g |
| Lauryl éther sulfate de sodium (C12/C1470/30) à 2,2 OE en solution aqueuse à 28 % vendu sous le nom d'Empicol ESB / 3 FL par Marchon | 10 g | 5 g |
| Polydiméthyl siloxane de PM 250 000 de viscosité 0,5 m²/s | 0,0 | 3 g |
| Iso-eicosane vendu sous le nom de Perméthyl 10 2 A par Créations Couleurs | 2 g | 0,0 |
| Microfibrilles cationiques de DS 0,6 | 1,5 g | 1,5 g |
| Colorants, parfum, conservateur eau qsp | 100 g | 100 g |

| **Exemple 21 : Shampooing** | % |
|---|---|
| Tensioactifs de l'exemple 6 du Brevet EP 699 472 | 8 |
| Microfibrilles de cellulose cationiques DS 0,6 | 1 |
| Peptides de blé | 0,5 |
| Phénonip | 0,3 |
| Colorant | qs |
| Parfum | qs |
| Eau | qsp 100 |

| **Exemple 22 : Tonic Lotion** | % |
|---|---|
| Tensioactifs de l'exemple 8 du brevet EP 699 472 | 1 |
| Microfibrilles de cellulose cationiques DS 0,6 | 0,5 |
| Eau bleuet | 5 |
| Phénonip | 0,5 |
| Colorant | qs |
| Parfum | qs |
| Eau | qsp 100 |

| **Exemple 23 : Gel douche** | % |
|---|---|
| Tensioactifs de l'exemple 7 du Brevet EP 699 472 | 10 |
| Tégobétaïne | 2 |
| Microfibrilles de cellulose cationiques DS 0,6 | 1,5 |
| NaCI | 1 |
| Peptide de blé | qs |
| Colorant | qs |
| Parfum | |
| Eau | qsp 100 |

| **Exemple 24 : Après shampooing** | **g** |
|---|---|
| Tensioactifs de l'exemple 6 du Brevet EP 699 472 | 4 |
| Heptaméthylnonane vendu sous le nom d'Arlamol HD par ICI | 10 |
| Microfibrilles de cellulose cationiques DS 0,6 | 1,6 |
| Colorant | qs |
| Parfum | qs |
| Eau | qsp 100 |

| **Exemple 25 : Après shampooing** | **g** |
|---|---|
| Lanette O (cetearylalcohol) | 4 |
| Heptaméthylnonane vendu sous le nom d'Arlamol HD par ICI | 10 |
| Microfibrilles de cellulose cationiques DS 0,6 | 1,6 |
| Colorant | qs |
| Parfum | qs |
| Eau | qsp 100 |

| **Exemple 26 : Après shampooing** | **g** |
|---|---|
| Lanette O (cetearyl alcohol) | 12 |
| Cetiol S N (Cetearyl Alcool Isononanoate) | 1 |
| Glycérol | 5 |
| Acide citrique | 0,5 |
| Emulgade (Lecithine) | 1 |
| Microfibrilles de cellulose cationiques DS 0,6 | 1,6 |
| Agent nacrant | 5 |
| Colorant | qs |
| Parfum | qs |
| Eau | qsp 100 |

### Exemple 27

Comparaison de propriétés floculantes des microfibrilles de cellulose cationiques et d'un polymère cationique de synthèse « C3427 L » commercialisé par CECA.

Le test de floculation, effectué sur une boue activée, consiste à centrifuger les boues après ajout du floculant et de récupérer le surnageant sur lequel on mesure la densité optique (turbidité).

On prépare 100 ml d'eau de rejet d'une station d'épuration. On mesure la matière sèche de la solution afin de déterminer la quantité de boues et de la matière en suspension à traiter. On ajoute à température ambiante 0,7 % de floculant par rapport à la matière sèche des boues. On centrifuge ensuite les boues et on récupère le surnageant. On détermine alors la turbidité du surnageant par mesure de la densité optique faite à 600 nm.

Les résultats sont mentionnés dans le tableau suivant :

| **Composés** | **Turbidité surnageant** |
|---|---|
| Témoin (boue sans floculant) | 10,2 |
| Polymère cationique C3427 L de CECA | 3,3 |
| Microfibrille cationique de DS 0,5 | 3,3 |
| Densité optique D.O. x 100 % (turbidité mesurée à 600 nm) | |

### Exemple 28

### Floculation d'une levure

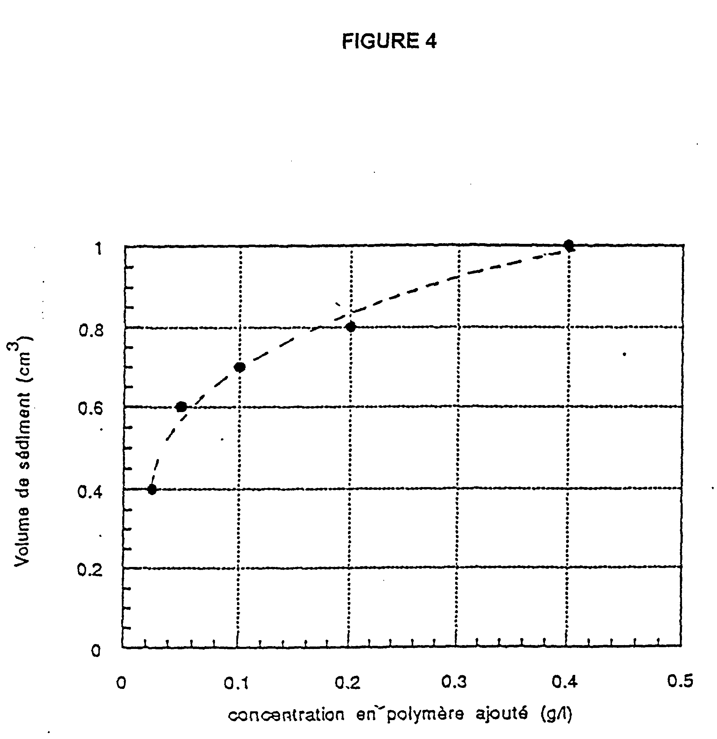

Des doses croissantes du polymère hydrosoluble (Cellulose cationique de DS 1) ont été ajoutées à une suspension de levure. La floculation est instantanée et le sédiment est d'autant plus compact que la quantité de polymère ajoutée est faible. Le surnageant est toujours clair, les levures peuvent donc être facilement séparées par centrifugation ou filtration. Les quantités de polymère ajoutées varient de 0,025 g/l à 0,4 g/l. Les résultats sont reportés sur la figure 4 ci-jointe.

### Exemple 29

### Activités antimicrobiennes des microfibrilles cationiques de DS 0,6.

La mise en évidence d'un effet bactériostatique et fongistatique et la détermination des concentrations inhibitrices minimales (CMI) pour chaque micro-organisme ont été réalisées par la méthode de dilution en milieu gélosé.

On incorpore avant stérilisation de quantités décroissantes de microfibrilles dans le milieu gélosé. On substitue une quantité d'agar par la même quantité de microfibrilles.

La CMI correspond à la concentration en produit la plus faible inhibant le développement microbien.

| Souches | | Cellulose native | | Microfibrilles cationiques DS 0,6 | |
|---|---|---|---|---|---|
| | | Concentrations testées (%) | CMI (% poids/poids) | Concentrations testées (%) | CMI (% poids/poids) |
| Bactéries Gram+ | Bacillus subtilis ATCC 6623 | 0,01 à 1 % | Croissance | 0,01 à 1 % | 0,4 % |
| Bactéries Gram- | P. aéruginosa ATCC9027 | 0,01 à 1 % | Croissance | 0,01 à 1 % | 1% |

Ainsi qu'on aura pu le comprendre, la présente invention permet d'obtenir des microfibrilles cationiques qui peuvent être deshydratées, mises sous forme de poudre par des stades opératoires simples et peu nombreux, sans traitement de lyophilisation ni d'ajout d'adjuvants, à partir de cellulose de son, de bois, de coton ou de cellulose issue de différents procédés d'obtention de la cellulose par les procédés papetier ou de toute autre nature.

On peut utiliser de la cellulose à partir de coproduits agricoles comme la bagasse, la pulpe de betterave et de chicorée, le son de blé, de riz, de maïs, la paille (blé, colza, riz), la luzerne, les rafles de maïs, les pulpes de fruits. La cellulose peut être purifiée selon le procédé de purification décrit dans le brevet américain n° 5.057.334.

Les produits obtenus selon le procédé de la présente invention peuvent être utilisés en tant qu'épaississants de boues de forage, qu'agents viscosifiants pour la récupération assistée du pétrole, qu'agents viscosifiants et rétenteurs d'eau dans le domaine de l'utilisation des ciments et des plâtres.

Du fait de leurs propriétés antimicrobiennes, ces mêmes produits trouvent des applications dans le domaine des objets tissés ou non tissés, à usage unique, qui doivent être des barrières pour les microorganismes tels que masques, fibres, etc.

## Revendications

1. Procédé de fabrication de microfibrilles de cellulose cationiques caractérisé en ce qu'il consiste à mettre des fibres de cellulose en contact avec un réactif cationique entre 20 et 90°C, en présence d'un agent alcalin, notamment de la soude et par le fait que la cellulose cationique obtenue est passée au moins une fois dans un homogénéisateur haute pression.

2. Procédé selon la revendication 1, caractérisé par le fait que le réactif cationique est de forme époxy et a pour formule (I) : 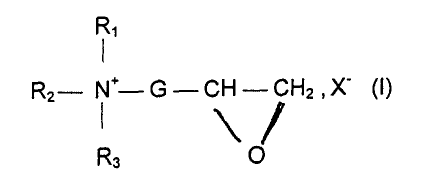 dans laquelle les substituants R₁, R₂, R₃ sont, de préférence, identiques et choisis parmi les radicaux méthyle et éthyle, l'un d'eux pouvant être un atome d'hydrogène, X⁻ représentant Cl⁻, Br⁻, I⁻ et G étant un espaceur hydrocarboné comptant de 1 à 6 atomes de carbone.

3. Procédé selon la revendication 1, caractérisé par le fait que le réactif cationique a pour formule (II) : 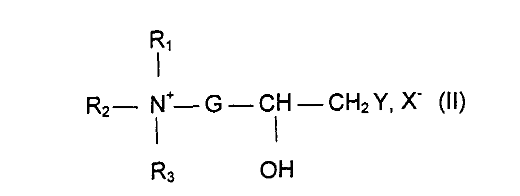 dans laquelle les substituants R₁, R₂, R₃ sont, de préférence, identiques et choisis parmi les radicaux méthyle et éthyle, l'un d'eux pouvant être un atome d'hydrogène, G étant un espaceur hydrocarboné comptant de 1 à 6 atomes de carbone et X⁻ représentant Cl⁻ , Bf⁻ , I⁻ et Y représentant Cl, Br, ou I.

4. Procédé selon l'une quelconque des revendications 1 à 3, caractérisé par le fait que la quantité d'agent alcalin mise en oeuvre est de 10⁻³ à 1, de préférence de 10⁻² à 10⁻¹ équivalent molaire par rapport à la cellulose lorsque le réactif cationique est celui de la formule (I), cette quantité est augmentée, lorsque le réactif cationique est celui de la formule (Il), d'une proportion correspondant à la neutralisation de l'agent de formule (II).

5. Procédé selon la revendication 1, caractérisé en ce qu'il consiste à mettre la cellulose cationique en suspension dans l'eau à une concentration comprise entre 0,5 et 10 %, de préférence de 1 à 5 % de matière sèche et par le fait que cette suspension est passée au moins une fois dans un homogénéisateur haute pression.

6. Microfibrilles de cellulose cationiques obtenues par le procédé selon l'une quelconque des revendications 1 à 5.

7. Microfibrilles selon la revendication 6, caractérisées par le fait qu'elles présentent un degré de substitution compris entre 0,1 et 0,7, sont majoritairement insolubles et ont un aspect de gel transparent.

8. Microfibrilles selon la revendication 6, caractérisées par le fait qu'elles présentent un degré de substitution supérieur ou égal à 0,7 et sont hydrosolubles.

9. Microfibrilles selon l'une quelconque des revendications 6 à 8, caractérisées par le fait qu'elles possèdent des activité microbiennes.

10. Application des microfibrilles, selon l'une quelconque des revendications 6 à 9, à la protection de produits contre des agressions microbiennes.

11. Application des microfibrilles, selon l'une quelconque des revendications 6 à 9, en tant qu'épaississant, agent stabilisateur et agent émulsifiant dans le domaine cosmétique.

12. Application des microfibrilles, selon l'une quelconque des revendications 6 à 9, en tant qu'épaississant, colloïde protecteur, et agent de suspension de pigments pour les peintures.

13. Application des microfibrilles, selon l'une quelconque des revendications 6 à 9, en tant qu'agent filmogène de couchage, adhésif pour surfaçage et de rétention de charges en papeterie.

14. Application des microfibrilles, selon l'une quelconque des revendications 6 à 9, en tant qu'agent épaississant, agent de suspension, liant, filmogène, stabilisateur pour émulsions dans les industries chimiques.

15. Application des microfibrilles, selon l'une quelconque des revendications 6 à 9, en tant qu'agent floculant pour les bactéries et les levures et dans le traitement des eaux.

16. Application des microfibrilles, selon l'une quelconque des revendications 6 à 9, en tant qu'agent viscosifiant et rétenteur d'eau dans le domaine de l'utilisation des ciments et des plâtres.

17. Application des microfibrilles, selon l'une quelconque des revendications 6 à 9 dans le domaine pétrolier, en tant qu'épaississant de boues de forage et agent viscosifiant pour la récupération assistée du pétrole.

## Patentansprüche

1. Verfahren zur Herstellung von kationischen Cellulose-Mikrofibrillen, dadurch gekennzeichnet, daß das Verfahren darin besteht, dass man Cellulosefasern bei 20-90°C mit einem kationischen Reagenz in Gegenwart eines alkalischen Mittels, insbesondere in Gegenwart von Natriumcarbonat, in Kontakt bringt und daß man die erhaltene kationische Cellulose mindestens einem Durchgang in einer Hochdruck-Homogenisiervorrichtung unterzieht.

2. Verfahren nach Anspruch 1, dadurch gekennzeichnet, daß das kationische Reagenz in Epoxyform vorliegt und die Formel (I) aufweist: 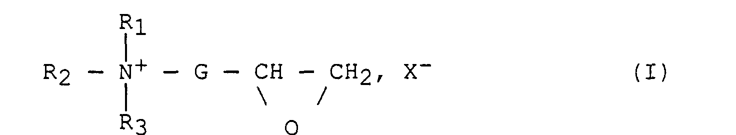 in der die Substituenten R₁, R₂ und R₃ vorzugsweise identisch sind und unter Methyl- und Ethylresten ausgewählt sind, wobei einer dieser Reste ein Wasserstoffatom bedeuten kann, X⁻ die Bedeutungen Cl⁻, Br⁻ oder I⁻ hat und G einen Kohlenwasserstoff-Abstandshalter mit 1 bis 6 Kohlenstoffatomen bedeutet.

3. Verfahren nach Anspruch 1, dadurch gekennzeichnet, daß das kationische Reagenz die Formel (II) aufweist: 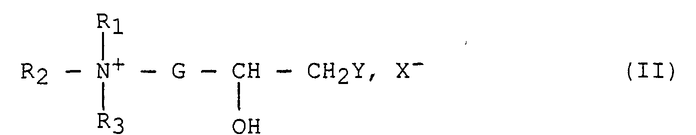 in der die Substituenten R₁, R₂ und R₃ vorzugsweise identisch sind und unter Methyl- und Ethylresten ausgewählt sind, wobei einer dieser Reste ein Wasserstoffatom bedeuten kann, G einen Kohlenwasserstoff-Abstandshalter mit 1 bis 6 Kohlenstoffatomen bedeutet, X⁻ die Bedeutungen Cl⁻, Br⁻ oder I⁻ hat und Y die Bedeutung Cl, Br oder I hat.

4. Verfahren nach einem der Ansprüche 1 bis 3, dadurch gekennzeichnet, daß die Menge des eingesetzten alkalischen Mittels 10⁻³ bis 1 und vorzugsweise 10⁻² bis 10⁻¹ Moläquivalente, bezogen auf die Cellulose beträgt, wenn das kationische Reagenz die Formel (I) aufweist, wobei diese Menge erhöht wird, wenn das kationische Reagenz die Formel (II) aufweist, und zwar um einen Anteil, der der Neutralisation des Mittels der Formel (II) entspricht.

5. Verfahren nach Anspruch 1, dadurch gekennzeichnet, daß es darin besteht, daß man die kationische Cellulose in Wasser in einer Konzentration von 0,5 bis 10% und vorzugsweise von 1 bis 5% Trockenmasse suspendiert und daß man die Suspension mindestens einem Durchgang in einer Hochdruck-Homogenisiervorrichtung unterzieht.

6. Kationische Cellulose-Mikrofibrillen, erhalten nach dem Verfahren gemäß einem der Ansprüche 1 bis 5.

7. Mikrofibrillen nach Anspruch 6, dadurch gekennzeichnet, daß sie einen Substitutionsgrad von 0,1 bis 0,7 aufweisen, überwiegend unlöslich sind und das Aussehen eines durchsichtigen Gels aufweisen.

8. Mikrofibrillen nach Anspruch 6, dadurch gekennzeichnet, daß sie einen Substitutionsgrad von 0,7 oder mehr aufweisen und wasserlöslich sind.

9. Mikrofibrillen nach einem der Ansprüche 6 bis 8, dadurch gekennzeichnet, daß sie mikrobielle Aktivitäten besitzen.

10. Verwendung der Mikrofibrillen nach einem der Ansprüche 6 bis 9 zum Schutz von Produkten gegen mikrobielle Angriffe.

11. Verwendung der Mikrofibrillen nach einem der Ansprüche 6 bis 9 als Verdickungsmittel, Stabilisierungsmittel und Emulgiermittel auf dem Gebiet der Kosmetik.

12. Verwendung der Mikrofibrillen nach einem der Ansprüche 6 bis 9 als Verdickungsmittel, Kolloidschutzmittel und Suspendiermittel für Pigmente in Anstrichmitteln.

13. Verwendung der Mikrofibrillen nach einem der Ansprüche 6 bis 9 als filmbildendes Beschichtungsmittel, Klebstoff für die Oberflächenbehandlung und Ladungsretentionsmittel bei der Papierherstellung.

14. Verwendung der Mikrofibrillen nach einem der Ansprüche 6 bis 9 als Verdickungsmittel, Suspendiermittel, Bindemittel, filmbildendes Mittel und Stabilisator für Emulsionen in der chemischen Industrie.

15. Verwendung der Mikrofibrillen nach einem der Ansprüche 6 bis 9 als Ausflockungsmittel für Bakterien und Hefen sowie bei der Wasserbehandlung.

16. Verwendung der Mikrofibrillen nach einem der Ansprüche 6 bis 9 als viskositätserhöhendes Mittel und als Wasserretentionsmittel auf dem Gebiet des Einsatzes von Zement- und Gipsprodukten.

17. Verwendung der Mikrofibrillen nach einem der Ansprüche 6 bis 9 auf dem Erdölgebiet als Verdickungsmittel für Bohrlochschlamm und als viskositätserhöhendes Mittel für die unterstützte Erdölgewinnung.

## Claims

1. A method of making microfibrils from cationic cellulose, characterised in that it consists in placing cellulose fibres in contact with a cationic reagent between 20 and 90°C in the presence of an alkaline agent such as soda and in that the resulting caticnic cellulose is conveyed at least once through a high-pressure homogeniser.

2. A method according to claim 1, characterised in that the cation reagent is epoxy in form and has the formula a (I) 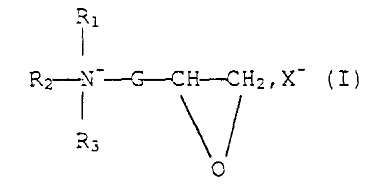 wherein the substituents R₁, R₂ and R₃ are preferably identical and chosen from among methyl and ethyl radicals, one of them optionally being a hydrogen atom, X⁻ denoting Cl⁻, Br⁻ or I⁻ and G being a hydrocarbon spacer containing 1 to 6 carbon atoms.

3. A method according to claim 1, characterised in that the cationic reagent has the formula (II): 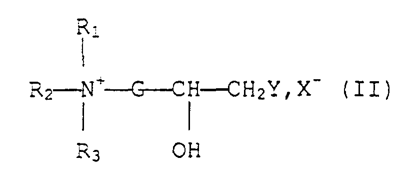 wherein the substituents R₁, R₂, R₃ are preferably identical and chosen from among methyl and ethyl radicals, one of them optionally being a hydrogen atom, G being a hydrocarbon spacer containing 1 to 6 carbon atoms, X⁻ denoting Cl⁻, Br⁻ or I⁻ and Y denoting Cl, Br or I.

4. A method according to any of claims 1 to 3, characterised in that the amount of alkaline agent used is 10⁻³ to 1, preferably 10⁻² to 10⁻¹ molar equivalent relative to cellulose when the cationic reagent has the formula (I) whereas when the cationic reagent has the formula (II) the quantity is increased by a proporation corresponding to neutralisation of the formula (II) agent.

5. A method according to claim 1, characterised in that it consists in suspending the cationic cellulose in water in a concentration between 0.5 and 10%, preferably 1 to 5% of dry matter and in that the suspension is conveyed at least once through a high-pressure homogeniser.

6. Microfibrils of cationic cellulose obtained by the method according to any of claims 1 to 5.

7. Microfibrils according to claim 6, characterised in that they have a degree of substitution between 0.1 and 0.7, are mainly insoluble and have the appearance of a transparent gel.

8. Microfibrils according to claim 6, characterised in that they have a degree of substitution greater than or equal to 0.7 and are soluble in water.

9. Microfibrils according to any of claims 6 to 8, characterised in that they have microbial activity.

10. Use of the microfibrils according to any of claims 6 to 9 for protection of products against corrosion by microbes.

11. Use of the microfibrils according to any of claim 6 to 9 as thickeners, stabilisers and emulsifiers in the cosmetics industry.

12. Use of the microfibrils according to any of claims 6 to 9 as thickeners, protective colloids and pigment suspensions agents in paints.

13. Use of the microfibrils according to any of claims 6 to 9 as film-forming coating agents, surfacing adhesives and adhesives for retention of fillers in stationery.

14. Use of the microfibrils according to any of claims 6 to 9 as thickeners, suspension agents, binders, film-forming agents or stabilisers for emulsions in the chemical industry.

15. Use of the microfibrils according to any of claims 6 to 9 as flocculating agents for bacteria and yeasts and in the treatment of water.

16. Use of the microfibrils according to any of claims 6 to 9 as viscosity-increasing and water-retaining agents in the use of cement and plaster.

17. Use of the microfibrils according to any of claims 6 to 9 in the petroleum industry as thickeners of drilling sludge and viscosity-increasing agents for assisted recovery of crude petroleum.
